# EUROPEAN PATENT APPLICATION

(11) **EP 4 806 363 A1**
(43) Date of publication of application: **16.09.2026**
(21) Application number: 25162491.2
(22) Date of filing: 10.03.2025
(51) Int. Cl.: A61B 6/40, A61B 6/00

(54) **ADAPTIVE CT DOSE MODULATION**

(71) Applicant: Koninklijke Philips N.V., 5656 AE Eindhoven (NL)
(72) Inventor: ERHARD, Klaus Alfred, Eindhoven (NL); DAERR, Heiner, Eindhoven (NL); BRENDEL, Bernhard Johannes, Eindhoven (NL); BONTUS, Claas, Eindhoven (NL); WIEGERT, Jens, Eindhoven (NL)
(74) Representative: Philips Intellectual Property & Standards

(57) **Abstract**

A mechanism for adjusting a tube current of a radiation source of a CT scanner while the CT scanner is actively acquiring a series of instances of CT projection data of a target volume. Each instance of acquired CT projection data is processed to determine a count metric representative of a number of counts in said instance of data. The count metric is compared to a predefined count range to determine whether or not to apply a current modifier to the tube current. In response to the count metric being outside the predefined count range, the tube current is either increased or decreased using the current modifier dependent on whether the count metric is above or below the predefined count range.

## Description

### FIELD OF THE INVENTION

The present invention relates to the field of computer tomography (CT), and in particular to methods of dose modulation in CT.

### BACKGROUND OF THE INVENTION

CT dose modulation, referring to the controlling of radiation (e.g., X-ray) dose or intensity delivered to a subject (e.g., a patient) during a CT scan, is often used to account for variations in anatomy and attenuation coefficients within the subject throughout the duration of the scan. Particularly, thicker and/or more attenuating parts of the subject's body are provided with a greater intensity of radiation than thinner and/or less attenuating parts. This results in less overall radiation exposure to the subject and somewhat consistent control of the signal-to-noise ratio in the measured signal.

CT dose modulation is also particularly important for photon-counting CT systems. Due to the direct conversion of photons into electrical signals and their ability to mask out most electrical noise, these CT systems are able to operate at lower intensity levels than conventional CT systems. However, this runs the risk of greatly reduced signal, or even photon starvation, from highly attenuating regions. On the other hand, detector over-exposure may lead to pulse pile-up effects which distort the measured energy spectrum. Maintaining the intensity of radiation received by the detector at an acceptable level is therefore critical for achieving high quality data.

The most common form of CT dose modulation is tube current modulation, where the current applied to the radiation source (e.g. X-ray tube) of the CT scanner is adjusted to control the output radiation flux/intensity. Estimations of the subject's anatomy (e.g., based on a CT scout scan) may be used to tailor the tube current modulation. However, this is prone to inaccuracies, particularly if a contrast agent is to be used during the CT scan which wasn't present in the estimation, potentially leading to moments of over- or under-exposure during the planned scan. There is thus a desire for improved methods of CT dose modulation.

### SUMMARY OF THE INVENTION

The invention is defined by the claims.

According to examples in accordance with an aspect of the invention, there is provided a computer-implemented method of modifying a tube current of a radiation source for a computer tomography, CT, scanner during acquisition of a series of instances of CT projection data of a target volume using the CT scanner. The computer-implemented method comprises, for each instance of CT projection data of a first set of the series of instances of CT projection data: receiving the instance of CT projection data from the CT scanner; processing the instance of CT projection data to determine a count metric responsive to a number of counts in the instance of CT projection data; determining whether the count metric is within a predefined count range; and, responsive to the count metric being outside the predefined count range, applying a current modifier to the tube current, wherein applying the current modifier to the tube current comprises increasing or decreasing the tube current using the current modifier.

The present disclosure provides an approach for modifying or updating a (tube) current applied to a radiation (e.g., X-ray) source of a CT scanner, thereby modulating a dose, intensity, or flux of radiation produced by the radiation source. Specifically, the proposed approach provides a mechanism for adjusting the current of the radiation source while acquiring CT projection data (using the CT scanner) of a target volume (e.g., a specific portion of a patient). The proposed technique thereby enables a received/measured radiation intensity or flux (as received by a detector of the CT scanner after passing through the target volume) to be maintained within a preferred range through modulation of the radiation source current.

The proposed approach makes use of (recently) acquired instances of CT projection data to monitor the measured intensity. Particularly, following acquisition of an (applicable) instance of CT projection data, said instance of data is processed to quantify the measured intensity in the form of a count metric that is representative of a number of counts in the instance of data, e.g., equal to a total number of counts, or equal to a number of counts over a particular portion of the data. The count metric is then compared to a preferred (i.e., predefined) count range to determine whether the measured intensity is at/within a preferred level. Accordingly, in response to the count metric being outside the count range, the tube current is modified in order to bring the count metric inside (or at least closer to) the count range. Specifically, the current is either increased or decreased, e.g., dependent on whether the count metric is below or above the count range, respectively.

When performed over the duration of a CT scan, the proposed technique allows for the measured intensity to be continuously monitored and adjusted (i.e., through modulation of the radiation source current) in order to keep it at a preferred level. In this way, the proposed method functions as a feedback loop to adaptively correct/regulate the radiation source current and account for changes in attenuation within the target volume.

In some examples, the predefined count range is defined by a predefined lower count limit and a predefined upper count limit. Accordingly, applying the current modifier comprises: in response to the count metric being below the predefined lower count limit, increasing the tube current using the current modifier; or, in response to the count metric being above the predefined upper count limit, decreasing the tube current using the current modifier.

The predefined count range may function as a preferred window for the count metric to stay within. Thus, in response to the count metric being below the window, the tube current is increased, i.e., to increase the count metric and bring it back within the window. Alternatively, in response to the count metric being above the window, the tube current is decreased, i.e., to decrease the count metric and bring it back within the window.

In some examples, the current modifier is determined responsive to: in response to the count metric being below the predefined lower count limit, a difference between the count metric and the predefined lower count limit; or, in response to the count metric being above the predefined upper count limit, a difference between the count metric and the predefined upper count limit.

The current modifier may therefore be determined based on how far away the count metric is from the predefined count range. For example, the current modifier may be greater (in magnitude) when the count metric is further outside of the predefined count range. This results in a greater change in the tube current, and thus a greater change in the count metric, therefore bringing the count metric closer to the predefined count range.

In some examples, the computer-implemented method further comprises, prior to applying the current modifier to the tube current: determining whether a modified tube current, being the tube current after applying the current modifier, is within a predefined current range; and, responsive to the modified tube current being outside the predefined current range, not applying the current modifier to the tube current.

The predefined current range may effectively define a safe or preferred operating range for the radiation source. Accordingly, it may be preferable for the tube current to not deviate from or go outside this range, even when the count metric is outside of the predefined count range.

In some examples, applying the current modifier to the tube current comprises applying the current modifier at a target time. It is recognized that processing of each instance of CT projection data (and any thereafter determination to apply a current modifier) may take a finite time, thus resulting in a delay in when the modifier is actually applied. Accordingly, it may be preferable to apply the current modifier at a later (i.e., target) time that is associated with the acquisition of an instance of CT projection data that is comparable to that used in determination of the current modifier.

For example, the target time may be associated with a time at which the radiation source has rotated 180° relative to an angular position of the radiation source when the instance of CT projection data was obtained by the CT scanner. This approach recognizes that instances of CT projection data acquired from opposite sides of the CT scanner represent effectively equivalent volumes of the subject (i.e., as they correspond to semi-equivalent projections of the target volume).

In some examples, the count metric is equal to a number of counts in a predetermined portion of the instance of CT projection data, said predetermined portion having been obtained by a predetermined subset of detector elements of a detector of the CT scanner.

The predetermined subset of detector elements may be specific for the instance of CT projection data (i.e., the subset may be different for each instance of CT projection data) and may be chosen based on a projection of a region-of-interest in the target volume on to the detector.

For example, the predetermined subset of detector elements may correspond to a specific subset of detector elements for the instance of CT projection data predicted to obtain CT projection data of a target region within the target volume. The target region may typically correspond to a region in the target volume for which increased image quality and/or signal-to-noise ratio is desired. For example, the target region may correspond to a particular organ of a patient. Accordingly, it may be preferable for the count metric to be based on counts/radiation received from the target region, e.g., to ensure the counts received from that region are at a preferred level.

Additionally, the predetermined subset of detector elements may be determined responsive to a predetermined model of the target volume and a projection angle associated with the instance of CT projection data. The predetermined model may correspond to a 3D digital model of the target volume describing a predicted distribution of attenuation coefficients contained within the target volume. This may have been determined from pre-acquired image data of the target volume.

In some examples, the predetermined model of the target volume is determined responsive to a CT scout scan of the target volume.

In some examples, the computer-implemented method further comprises: responsive to the count metric being within the predefined count range, determining whether the tube current is different to a predetermined tube current, wherein the predetermined tube current is determined responsive to the predetermined model of the target volume; and, responsive to the tube current being different to the predetermined tube current, applying a second current modifier to the tube current, wherein the second current modifier is proportional to a difference between the tube current and the predetermined tube current.

In this context, the predetermined tube current may correspond to a planned tube current (e.g., based on the predetermined model) for obtaining a preferred/planned radiation intensity or flux on the detector of the CT scanner. Thus, while the count metric is within the predefined count range, this mechanism may help to keep the tube current close to the predetermined tube current by applying the second current modifier.

In some examples, the computer-implemented method further comprises: responsive to the count metric being within the predefined count range, determining whether the count metric is different to a predetermined count metric, wherein the predetermined count metric is determined responsive to a predetermined tube current and the predetermined model of the target volume; and, responsive to the count metric being different to the predetermined count metric, applying a third current modifier to the tube current, wherein the third current modifier is proportional to a difference between the count metric and the predetermined count metric.

In this context, the predetermined count metric may be associated with a planned or preferred number of counts in the instance of CT projection data (i.e., based on the predetermined model). Thus, while the count metric is within the predefined count range, this mechanism may help to keep the count metric close to the predetermined count metric by controlling the tube current using the third current modifier (e.g., increasing or decreasing the tube current to bring the count metric closer to the predetermined count metric).

In some examples, the CT scanner is a photon-counting CT scanner.

There is also provided a computer program product comprising computer program code means which, when executed on a computing device having a processing system, cause the processing system to perform all of the steps of any of the previously disclosed methods.

There is further provided a processing system for modifying a tube current of a radiation source for a computer tomography, CT, scanner during acquisition of a series of instances of CT projection data of a target volume using the CT scanner. The processing system is configured to, for each instance of CT projection data of a first set of the series of instances of CT projection data: receive CT projection data of the target volume from the CT scanner; process the CT projection data to determine a count metric responsive to a number of counts in the CT projection data; determine whether the count metric is within a predefined count range; and, responsive to the count metric being outside the predefined count range, apply a current modifier to the tube current, wherein applying the current modifier to the tube current comprises increasing or decreasing the tube current using the current modifier.

There is further provided a CT system comprising the previously disclosed processing system and the CT scanner.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a CT system;
Fig. 2 shows a flowchart illustrating a proposed method;
Fig. 3 schematically illustrates illumination of a target region by a CT scanner; and
Fig. 4 illustrates an example of a time-variable tube current and count metric.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Throughout this disclosure, ordinal numbers (e.g. "first", "second" and so on) have been used purely to distinguish different elements from one another for the sake of clarity, and reference to a non-"first" (e.g. "second" or "third") element does not necessitate that a "first" element be present. The skilled person would be capable of relabeling any such elements as appropriate (e.g. relabeling a "second" element as a "first" element if only the second element is present).

The invention provides a mechanism for adjusting a tube current of a radiation source of a CT scanner while the CT scanner is actively acquiring a series of instances of CT projection data of a target volume. Each instance of CT projection data of a first set of the acquired series of instances is processed to determine a count metric representative of a number of counts in said instance of data. The count metric is compared to a predefined count range to determine whether or not to apply a current modifier to the tube current. In response to the count metric being outside the predefined count range, the tube current is either increased or decreased using the current modifier dependent on whether the count metric is above or below the predefined count range.

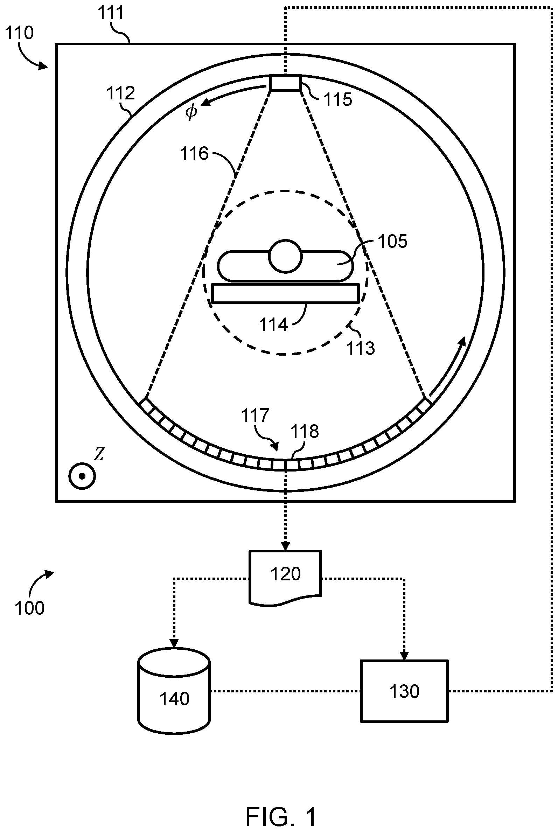

Fig. 1 illustrates a CT system 100 in which proposed embodiments may be employed for improved contextual understanding.

The CT system 100 comprises a CT scanner 110 for acquiring a CT projection dataset of a target volume. The target volume may typically correspond to a part/portion of a subject 105, e.g., a patient. Particularly, acquisition of the CT projection dataset may be for diagnostic purposes, e.g., for generating an image of the subject's internal anatomy to aid in diagnosing a medical condition. In some instances, the target volume may correspond to the entirety of the subject 105, i.e., the whole of the subject's body.

The illustrated CT scanner 110 comprises a stationary gantry 111 and a rotating gantry 112 supported by the stationary gantry 111. The rotating gantry 112 is configured to rotate (relative to the stationary gantry 111) about a longitudinal axis Z around an examination region 113. The CT scanner 110 further comprises a support 114 for supporting the subject 105 such that (at least) a portion of the subject 105 is within the examination region 113.

A (penetrating) radiation source 115, such as an X-ray tube, is arranged on the rotating gantry 112 for rotation therewith. The radiation source 115 is configured to produce a beam of radiation 116 that passes through the examination region 113 during rotation of the rotating gantry 112, thereby illuminating the portion of the subject 105 over a plurality of projection angles *ϕ*. The beam of radiation 116 may be shaped (e.g., using a collimator provided in, or in front of, the radiation source 115) into a cone or fan-shaped beam. The beam of radiation 116 may also be substantially monochromatic, e.g., through use of a suitable filter provided in, or in front of, the radiation source 115.

The CT scanner 110 also comprises a detector 117 arranged on the rotating gantry 112 for rotation therewith. The detector 117 is positioned opposite the radiation source 115 such that it receives a substantial amount of the beam of radiation 116 after passing through the examination region 113. The detector 117 comprises a plurality of detector elements 118 for detecting the received radiation, e.g., through the conversion of (X-ray) photons into electrical signals. Specifically, each detector element 118 may detect/measure a respective portion of the radiation received by the detector 117. The plurality of detector elements 118 may be arranged as an array, such as a 1D or 2D array. The detected radiation is subsequently used to form the CT projection dataset which (from rotation of the rotating gantry 112) is acquired from the portion of the subject 105 over the plurality of projection angles *ϕ*.

During rotation of the rotating gantry 112, the support 114 may be translated or moved parallel to (i.e., along) the longitudinal axis of rotation Z, thereby changing the portion of the subject 105 within the examination region (and thus the portion from which the CT projection dataset is acquired). By controlling the translation of the support 114, the CT projection dataset may be acquired from a desired portion/volume of the subject (e.g., the target volume). In certain examples, said translation of the support 114 may be continuous (corresponding to helical CT scanning) or discrete/iterative (corresponding to axial/step-and-shoot CT scanning). Other mechanisms for moving the rotating gantry 112 and support 114 with respect to each other (along the longitude axis of rotation) are known, such as translating or moving the rotating gantry 112 as it rotates.

During operation (i.e., when acquiring the CT projection dataset) the detector 117 (or more specifically the detector elements 118 of the detector 117) is/are sampled at predetermined time intervals, i.e., predetermined sampling intervals. The CT projection dataset (corresponding to e.g., electrical signals produced by each detector element 118) thus comprises a plurality of discrete data values or data entries, hereby referred to as projection values. Each projection value of the dataset is associated with a time (interval) at which the projection value was measured or sampled (or equivalently a projection angle *ϕ* at which the projection value was measured or sampled) and a detector element 118 that measured the projection value.

Each projection value may also be associated with a longitudinal position Z of the support 114 and/or subject 105 (and thus of the target volume), however this information may instead be absorbed/contained within or represented by the time parameter (or projection angle parameter when extended beyond 360°).

The CT projection dataset thus comprises a multi-dimensional (e.g., 2D, 3D, 4D) dataset, where each pixel/voxel is associated with (and has a value given by) a respective projection value. Particularly, the dataset may have one or more dimensions each associated with a dimension of the detector 117 (e.g., a row-dimension and a column-dimension when the detector 117 comprises a 2D array of detector elements 118) and a time dimension associated with a time parameter (which may also be defined in terms of a projection angle parameter and a longitudinal position parameter).

To provide further context, each projection value is further associated with a path through the examination region 113 and/or the target volume, being the (approximate) path taken by each photon that contributed towards the projection value. Specifically, the path for a particular projection value corresponds to a straight line between the radiation source 115 and the detector element that measured the projection value. Accordingly, each projection value is representative of parts of the target volume along the associated path (or more precisely representative of properties of the parts of the target volume, e.g., attenuation properties).

Each projection value typically has an associated value/magnitude (or is itself the associated value) corresponding to a (e.g., electrical) signal produced by the associated detector element, said signal being representative of an amount/quantity of radiation (e.g., counts, flux, intensity, etc.) detected by the associated detector element over the predetermined sampling interval.

The CT projection dataset is typically acquired as a (time) series of instances 120 of CT projection data. Each instance 120 of CT projection data corresponds to data acquired over a respective time interval of the total time interval/period used to acquire the CT projection dataset, i.e., the total time interval/period of the CT scan. Each instance 120 of CT projection data thus corresponds to a respective/distinct data portion of the resulting CT projection dataset.

In some examples, each instance 120 of CT projection data is associated with a respective sampling of the detector 117. That is, each instance 120 of CT projection data is captured at/over a respective predetermined sampling interval of the detector 117. Put another way, the series of instances 120 of CT projection data is acquired at the same rate as the sampling rate of the detector 117.

Each instance 120 of CT projection data thus comprises a (single) projection value for each detector element 118. Each instance 120 of CT projection data may also be associated with a respective projection angle *ϕ* and longitudinal position Z pair, being the angular position of the radiation source 115 and the longitudinal position of the support 114, respectively, when the instance 120 of CT projection data is/was captured.

In other examples, each instance 120 of CT projection data may comprise data from multiple samplings of the detector 117. Particularly, each instance 120 of CT projection data may comprise data acquired over multiple projection angles *ϕ*, e.g., over a partial or full rotation of the radiation source 115.

The CT system 100 further comprises a processing system 130. Following acquisition of an instance 120 of CT projection data, the CT scanner 110 may provide the instance 120 of CT projection data to the processing system 130 for processing. Particularly, the processing system 130 may process the instance 120 of CT projection data for the purposes of reconstructing a CT image representing the target volume (e.g., using a back projection technique). The processing system may also process the instance 120 of CT projection data for the purposes of performing a proposed method, as will be later disclosed.

When reconstructing the CT image, the processing system 130 may process each instance 120 of CT projection data when received. In other examples, the processing system 130 may store each received instance 120 of CT projection data in a local memory (e.g., RAM, PROM, EPROM, EEPROM, etc.) in order to collate a larger portion of data before processing said data. For example, the processing system 130 may wait until it has collated the full series of instances of CT projection data (i.e., obtained the CT projection dataset) before reconstructing the CT image.

The CT system 100 may also comprise a memory 140. Following acquisition of an instance 120 of CT projection data, the CT scanner 110 may provide the instance 120 of CT projection data to the memory 140 for storage. This may be instead of, or in addition to, providing the instance 120 of CT projection data to the processing system 130. Each acquired instance 120 of CT projection data may be stored together in the memory 140 as a sequence/series (e.g., concatenated as a single file) in order to obtain the CT projection dataset.

The memory 140 may take the form of a local memory (e.g., HDD, SSD, etc.) or an external (i.e., non-local) memory or database (e.g., external server, cloud server, etc.).

The processing system 130 is in communication with the memory 140 to facilitate data transfer/retrieval. For instance, the processing system 130 may retrieve one or more instances of CT projection data (e.g., the full CT projection dataset) from the memory 140 in order to reconstruct the CT image. The processing system 130 may also provide data/information (e.g., the CT image) to the memory 140 for storage.

The processing system 130 may also retrieve other information/data from the memory 140 for the purposes of performing a proposed method, as will be later disclosed.

The processing system 130 is also in communication with the radiation source 115 of the CT scanner 110. The processing system 130 may control aspects of the radiation source, e.g., a tube current, as will be expanded on later.

The CT scanner 110 is preferably a photon-counting CT scanner. In particular, the detector 117 may preferably comprise a photon-counting detector for detection of individual (X-ray) photons. In such examples, each detector element 118 comprises a semiconducting material for conversion of received radiation (e.g., X-ray photons) into charge carriers (e.g., electron-hole pairs) and a respective anode for attraction of the generated charge carriers which are measured as an electric pulse. Each measured electric pulse is counted as a single received photon, where the magnitude of the pulse is proportional to the energy of said photon. Accordingly, such detectors are able to detect the number of photons transmitted through a target volume (and also the energy of said photons) which is indicative of the composition and attenuation of the target volume.

A projection value acquired by a detector element of a photon-counting CT scanner may typically be representative of a distribution of measured photon energies. Specifically, each measured photon may be sorted in one of a series of energy bins depending on its measured energy, the projection value comprising the number of photons in each bin. Each projection value is thus indicative of the number of photons received by a detector element over a particular sampling interval, and (at least approximately) indicative of the energy distribution of said photons.

The radiation source 115 may typically comprise an X-ray tube or another radiation source that produces radiation through acceleration/deceleration of a beam of electrons. For example, in the case of an X-ray tube, the beam of electrons is accelerated through an electric field onto a metal anode, causing (at least some of) the electrons to rapidly decelerate and produce X-ray photons. The beam of electrons is associated with a current, hereby referred to as a tube current, which is proportional to the charge density and speed of the electron beam. The amount of radiation produced by the radiation source 115 (e.g., flux, intensity, etc.) is proportional to the tube current, where a greater tube current (corresponding to an electron beam comprising more and/or faster electrons) results in a greater amount of radiation. Thus, the amount of radiation produced by the radiation source 115 (and by extension the dose of radiation received by the subject 105 and/or the target volume) is controllable through controlling the tube current.

Physical mechanisms for controlling/changing a tube current, e.g., changing the temperature of a cathode filament, or changing the strength of the electric field, will be readily apparent to the skilled person.

Controlling tube current as a function of time, referred to as tube current modulation, is commonly employed to control the dose of radiation received by a target volume during a CT scan. This is used to account for variations in attenuation (within the target volume) which may otherwise result in differing amounts of radiation being transmitted through the target volume. Instead, tube current modulation is able to provide for a more consistent amount of transmitted radiation, and thus a more consistent amount of detected radiation. As well as reducing the overall radiation dose received by the target volume, this also provides a more stable signal-to-noise ratio, reducing the amount of noise, graining, and other artifacts, in the final/resulting CT image.

In conventional (i.e., energy-integrated) CT scanners, greater radiation intensity/flux generally results in a higher quality CT image due to a better overall signal-to-noise ratio. However, in photon-counting CT scanners, greater radiation intensity/flux may result in signal degradation/distortion due to so-called pulse pile-up effects. This is where two or more photons are detected by a single detector element in a short timeframe, such that their resulting electric pulses overlap and cannot be individually resolved. The result is that the detected photons are registered as a single higher energy photon, skewing the measured energy spectrum. This is made worse at higher radiation intensities/photon fluxes, where more photons reach the detector in a short space of time, increasing the likelihood of pulse pile-up effects.

Tube current modulation therefore provides significant advantages in photon-counting CT. Particularly, it is able to prevent the amount of measured radiation (i.e., counts) both dropping too low and rising too high. In other words, tube current modulation may be utilized to maintain the measured counts within a preferred window for increased image/data quality.

A tube current modulation protocol/program is typically pre-planned or pre-programmed, i.e., how the tube current will vary is known prior to performing the CT scan. This is most often achieved through (pre-) acquiring test or scout data from the target volume, being low-quality data indicative of the attenuation and/or composition of the target volume. For example, a CT scout scan (i.e., a fast, low-dose CT scan) is commonly performed on a target volume prior to performing a clinical CT scan. Though typically performed to enable more accurate positioning of the target volume for the clinical scan, the CT scout scan also allows for planning of the tube current modulation protocol based on the estimated target volume composition.

Planning a tube current modulation protocol typically comprises determining a time-dependent tube current describing the radiation source tube current as a function of time. Such planning may take account of the rotation speed of the CT scanner and the longitudinal displacement speed of the target volume, thereby accounting for which part of the target volume is being imaged at different times of the CT scan.

During a tube current modulation protocol, the tube current of the radiation source may be controlled by a processing system (e.g., the processing system 130 of Fig. 1). In particular, the processing system may be provided with the (predetermined) time-dependent tube current, which indicates (to the processing system) how the tube current should vary over the CT scan. The processing system may then control the tube current accordingly.

The present disclosure recognizes that planning of the tube current modulation may not always be accurate. Particularly, the low-quality of the scout data may cause inaccurate estimations of the target volume, leading to deviations from an intended dose level or measured intensity during the clinical CT scan. Additionally, clinical CT scans are commonly performed in conjunction with an injected contrast agent to enhance the visibility of certain regions in the resulting CT image. However, contrast agents are usually not administered in CT scout scans. Thus, their effects are only estimated during planning of the tube current modulation, leading to further inaccuracies.

The present disclosure proposes a mechanism for modifying a tube current of a radiation source during a CT scan. The proposed approach makes use of instances of CT projection data acquired during the CT scan to monitor a received/measured intensity (in particular a number of counts) as measured by a detector. If the measured intensity deviates from a preferred range, the tube current is adjusted to bring the intensity back towards the preferred range. In this way, when performed over the duration of a CT scan, the tube current is controlled to keep the measured intensity at/within a desired level.

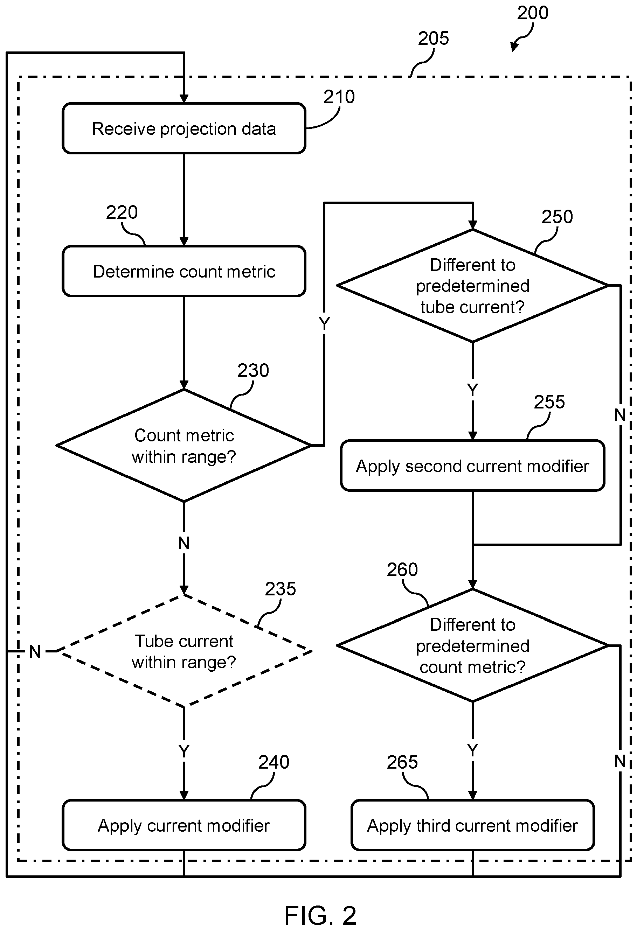

Fig. 2 is a flowchart illustrating a computer-implemented method 200 for modifying a tube current of a radiation source during a CT scan, i.e., during acquisition of a series of instances of CT projection data of a target volume using a CT scanner. In particular, the method 200 may be performed by the processing system 130 (Fig. 1) of the CT system 100.

It will be appreciated that, in the absence of the method 200 (or at least prior to performing the method), the tube current of the radiation source may possess a default/nominal value or be in a default/nominal state. Particularly, said default value or state may be in accordance with a (preset) configuration/program/protocol for the tube current, e.g., as set by a user at/before the start of the CT scan. For instance, the tube current may be programmed to take a constant value, or to follow a time-dependent tube current. The proposed method thus acts to (at least temporarily) override this behavior, e.g., in order to achieve certain preferable or favorable scanning conditions.

The method 200 comprises performing a monitoring process 205 for each instance of CT projection data of a first set of the series of instances of CT projection data, i.e., a set of instances of CT projection data comprising at least some of the instances of CT projection data acquired by the CT scanner. The first set thus indicates which instances of the series of instances of CT projection data are to be processed according to the monitoring process 205.

In this context, it will be appreciated that the first set is comprised by the series of instances of CT projection data. That is, the first set is not itself a separate collection of instances of CT projection data, but rather forms part of the series of instances of CT projection data.

In some examples, the first set comprises less instances of CT projection data than the series of instances of CT projection data. For example, the first set may comprise every other instance of the series of instances of CT projection data, or every n-th instance. Processing only some of the instances of CT projection data acquired by the CT scanner advantageously reduces computation costs.

As another example, the instances of CT projection data comprised by the first set may not be evenly spaced in time. For instance, the first set may be more densely sampled (i.e., comprise more instances of CT projection data within a given time interval) at times when there is expected to be greater variation in the tube current, e.g., as indicated by a (predetermined) time-dependent tube current. Thus, certain times of the CT scan may be monitored more precisely.

In other examples, the first set comprises all of the instances of CT projection data of the series of instances of CT projection data. In other words, the first set is the series of instances of CT projection data.

The monitoring process 205 is preferably performed immediately after acquisition of each instance of CT projection data of the first set, or at least performed in close temporal proximity to the time of acquisition of each instance of CT projection data of the first set. In particular, this is so that the outcome of the monitoring process 205 may affect later instances of CT projection data.

The monitor process 205 is performed once for each instance of CT projection data of the first set. This may comprise performing the monitoring process 205 for each instance of CT projection data (of the first set) in series, i.e., performing the monitoring process 205 for a particular instance before performing the monitoring process 205 for a next instance. In other examples, the monitoring process 205 may be performed for multiple (i.e., more than one) instances of CT projection data at once, i.e., performed in parallel. In other words, different instances/iterations of the monitoring process 205 may be performed concurrently.

The monitoring process 205 comprises a step 210 of receiving an instance of CT projection data from the CT scanner. In particular, step 210 comprises receiving the instance of CT projection data for which the present iteration of the monitoring process 205 is to be performed.

For use in later disclosures, the instance of CT projection data received in step 210 may be referred to as the present instance of CT projection data, or simply as the instance of CT projection data. Particularly, instances of CT projection data which do not correspond to the instance of CT projection data for the present iteration of the monitoring process 205 will be explicitly distinguished from the present instance of CT projection data.

When the method 200 is performed by the processing system 130 (Fig. 1), step 210 comprises the processing system 130 receiving the instance 120 of CT projection data from the CT scanner 110. This may comprise the CT scanner 110 transferring the instance 120 of CT projection data to the processing system 130, e.g., in response to acquiring the instance 120 of CT projection data. In other examples, this may comprise the processing system 130 retrieving the instance 120 of CT projection data from the CT scanner 110, e.g., from a memory, buffer, or cache of the CT scanner.

The monitoring process 205 further comprises a step 220 of processing the (received) instance of CT projection data to determine a count metric responsive to a number of counts in the instance of CT projection data. In this context, the count metric is representative of (e.g., quantifies) at least some of the counts in the instance of CT projection data. For example, the count metric may be representative of the number of counts in the entirety of the instance of CT projection data, or only in a portion of the instance of CT projection data. Thus, the count metric may be embodied as a number/value, e.g., equal to the represented number of counts.

The number of counts corresponds to the number of (X-ray) photons measured by the detector of the CT scanner during acquisition of the instance of CT projection data, i.e., over an acquisition period. The number of counts is therefore indicative of an amount of radiation (e.g., number of photons) transmitted through the target volume during the acquisition period.

Where the CT scanner is a photon-counting CT scanner, the number of counts in the instance of CT projection data may be determined directly from the projection values of the instance of CT projection data. Specifically, this may comprise summing together (at least some of) the projection values of the instance of CT projection data, or more precisely the number of photons represented by each projection value.

In other examples, i.e., where the CT scanner is not a photon-counting CT scanner, the number of counts may be estimated from the projection values of the instance of CT projection data.

The count metric may thus be determined through processing the projection values of the instance of CT projection data, e.g., through extraction and summation of particular (projection) values.

As will become evident later, the count metric is indicative of whether an amount of radiation received by a detector of the CT scanner is at a preferred level, e.g., for achieving a preferred image quality or signal-to-noise ratio in a resulting CT image.

It is recognized that not all parts of the detector will receive equal or equivalent amounts of radiation, e.g., due to variations in attenuation in the target volume, the target volume only occupying a portion of the full examination region, etc. Thus, it may be preferable to base the count metric only on radiation received by a part of the detector, i.e., a subset of detector elements of the detector.

Accordingly, in some examples, the count metric is determined from a predetermined portion of the instance of CT projection data, said predetermined portion comprising data obtained by a predetermined subset of detector elements of the detector. Specifically, the count metric may be set equal to the number of counts in the predetermined portion of the instance of CT projection data.

It will be appreciated that each detector element of the detector is associated with one or more projection values in the instance of CT projection data (i.e., being the detector element that measured the projection value). Thus, each detector element directly maps to a part of the instance of CT projection data. The predetermined portion of the instance of CT projection data is therefore directly identifiable from the predetermined subset of detector elements.

The predetermined subset of detector elements may typically correspond to a subset of detector elements expected to obtain data that is of more significance to the final/resulting CT image than other detector elements. For instance, the subset of detector elements may exclude (i.e., not include) detector elements towards the edge/periphery of the detector that are not expected to receive any radiation transmitted through the target volume.

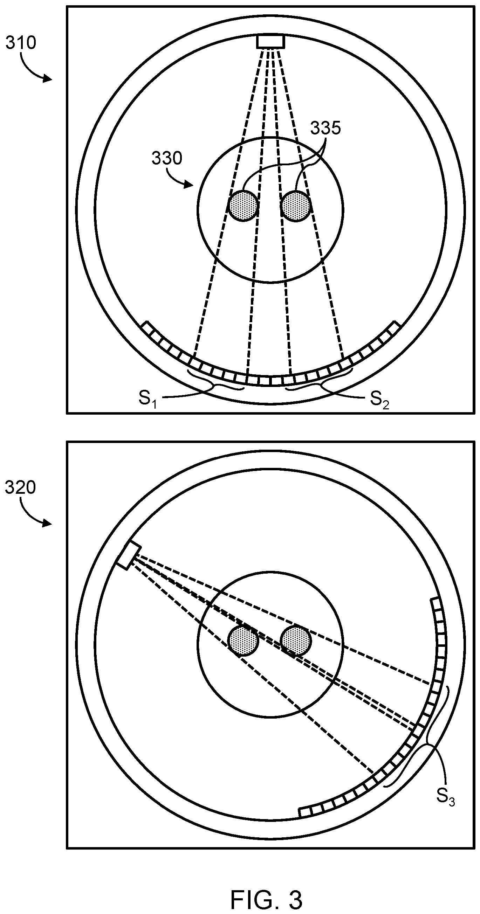

In accordance with a preferred example, the predetermined subset of detector elements corresponds to a subset of detector elements predicted to obtain CT projection data of a target region within the target volume. This embodiment is conceptualized in Fig. 3 which illustrates acquisition of an instance of CT projection data from a target volume 330 at two different times 310 and 320, each corresponding to a different (i.e., respective) angular position of a radiation source.

The target volume 330 comprises a target region 335 which corresponds to a particular region of interest in the target volume 330, e.g., for diagnosing a medical condition. For example, where the target volume 330 is a portion of a subject, the target region 335 may correspond to a particular organ of the subject. In particular, Fig. 3 depicts the target region 335 as two separate/distinct regions, which could represent a pair of lungs. Of course, in other examples, the target region could comprise a single continuous region of the target volume.

The radiation source illuminates the target region 335 with a beam of radiation which causes a projection of the target region 335 onto a detector. Accordingly, for a given angular position of the radiation source, only certain detector elements of the detector will receive radiation that has been transmitted through the target region 335. By extension, only data captured (i.e., radiation measured) by those detector elements will contribute towards a representation of the target region in a final CT image.

For example, at time 310, only subsets S₁ and S₂ of the detector elements receive radiation from the target region 335. Thus, when reconstructing a CT image using the instance of CT projection data acquired at time 310, only portions of the instance of CT projection data comprising data acquired by subsets S₁ and S₂ will contribute to a representation of the target region.

It may be desirable for the portion of the resulting CT image representing the target region to have a sufficiently high image quality or signal-to-noise ratio, e.g., due to the clinical importance of the target region. Thus, it may be preferable for the amount of radiation received from the target region to be at a particular level predicted to achieve the desired image quality.

To achieve this, the count metric may be determined from the data captured by the subset of detector elements that receives radiation from the target region 335 (e.g., subsets S₁ and S₂ at time 310). In this way, the count metric is only sensitive to the amount of radiation received by said subset of detector elements, and may thus be used to more effectively control this amount of radiation. Put another way, this approach gives higher priority to the image quality of the representation of the target region compared to other parts of the final CT image.

In practice, the subset of detector elements will preferably be predetermined (i.e., known before performing the CT scan) so that determination of the count metric is automatic. Therefore, the position and shape of the target region in the target volume should desirably be known prior to performing the CT scan. In particular, a (predetermined) model or estimation of the target volume (e.g., describing the composition of the target volume) may be obtained prior to performing the CT scan.

The predetermined model may be based on scout data acquired prior to the CT scan. In particular, the predetermined model may be determined responsive to a CT scout scan of the target volume.

Hereafter, any predetermined subset of detector elements is referred to as a sentinel detector. Thus, each sentinel detector corresponds to a predetermined portion of a detector comprising one or more detector elements.

The predetermined model may be used to determine the sentinel detector. For example, the sentinel detector may be determined by simulating projection of the target region (as contained in the predetermined model) onto a detector, similar to that demonstrated in Fig. 3.

The sentinel detector for a particular instance of CT projection data is thus dependent on the predetermined model and the projection angle at which the instance of CT projection data was acquired. In particular, this combination defines how the target region is projected on to the detector, and thus what detector elements receive radiation from the target region.

The present disclosure further recognizes that the subset of detector elements that receives radiation from the target region may vary over the duration of the CT scan (i.e., change as a function of time or projection angle) dependent on the shape and position of the target region. This is demonstrated in Fig. 3 where, at time 310, subsets S₁ and S₂ receive radiation from the target region 335, whereas at time 320, subset S₃ receives radiation from the target region 335.

In other words, as the radiation source rotates around the target volume, the projection of the target region onto the detector may change, thereby altering which detector elements receive radiation from the target region. Thus, each instance of CT projection data may be associated with a different sentinel detector, i.e., a specific/respective subset of detector elements for the instance of projection data. Put another way, the sentinel detector used to determine a count metric may change over the duration of a CT scan to reflect changes in the projection of the target region onto the detector.

Thus, in practice, a series of sentinel detectors may be determined prior to performing the CT scan, each sentinel detector being associated with a respective projection of the target region. More specifically, each sentinel detector may be associated with a projection angle and a longitudinal position of the target region, which thereby define the projection of the target region onto the detector. Accordingly, each acquired instance of CT projection data may be matched with a respective sentinel detector (which dictates what parts of the instance of CT projection data to use when determining a count metric) based on the projection angle (and longitudinal position) associated with the instance of CT projection data.

The monitoring process 205 further comprises a step 230 of determining whether the count metric is within a predefined count range.

The predefined count range corresponds to a preferred range for the count metric to lie within, representative of a preferred amount of radiation (e.g., number of photons) to be received by the detector of the CT scanner, e.g., for achieving a preferred image quality or signal-to-noise ratio in a resulting CT image.

The predefined count range may be defined by an associated threshold (e.g., a value). Upon breaching the threshold (i.e., being above or below the threshold), the count metric may be said to be within (or not within) the predefined count range.

For example, the threshold may represent a minimum number of counts required to achieve a sufficient signal-to-noise ratio in a resulting CT image. Thus, while the count metric is above (i.e., greater than) the threshold, it may be said to be within the predefined count range.

According to a preferred example, the predefined count range may be defined by a predefined lower count limit and a predefined upper count limit. In other words, the predefined count range may be a predefined count window. Thus, while the count metric is above the predefined lower count limit and below the predefined upper count limit, it may be said to be within the predefined count range. However, if the count metric were to be below the predefined lower count limit or above the predefined upper count limit, it may be said to be outside the predefined count range.

As explained previously, the use of a predefined count window is particularly advantageous in photon-counting CT.

If the count metric is determined to be outside of the predefined count range, this is interpreted to mean that the present amount of radiation received by (at least part of) the detector, or at least the amount received during acquisition of the instance of CT projection data, is not at a preferred level. Thus, it would be preferable to change (e.g., increase or decrease) the amount of radiation being provided to the target volume (i.e., the amount of radiation emitted by the radiation source) so that a different (e.g., more preferable) amount of radiation is subsequently received by the detector.

Accordingly, the monitoring process 205 comprises a step 240 of modifying the tube current of the radiation source by applying a current modifier to the tube current. Specifically, applying the current modifier to the tube current comprises increasing or decreasing the tube current using the current modifier, e.g., dependent on whether the amount of radiation received by the detector needs to be increased or decreased. The monitoring process 205 performs this step in response to the count metric being outside the predefined count range.

In practice, application of the current modifier may comprise controlling the radiation source to increase or decrease its tube current by an amount indicated by the current modifier. For example, the current modifier may define (or itself be) an electrical current value or offset of constant magnitude. Thus, applying the current modifier may comprise changing the tube current by the current offset defined by the current modifier, e.g., adding or subtracting the current offset to/from the tube current.

As another example, the current modifier may be embodied as a physical electrical current that is applied to the radiation source in addition to the tube current. Thus, the total (tube) current present in the radiation source may be the sum of the tube current and the current modifier.

As a further example, the current modifier may comprise a scaling factor to be applied to the tube current. Thus, applying the current modifier may comprise multiplying the tube current by the current modifier. Accordingly, the tube current may be scaled up (i.e., increased) or scaled down (i.e., decreased) dependent on whether the current modifier is less than or greater than 1.

For use in later disclosure, the (total) tube current present in or applied to the radiation source after applying the current modifier (or what would be present in the radiation source after applying the current modifier) is hereby referred to as the modified tube current.

The current modifier may be applied to the tube current (substantially) instantaneously. **In** other words, the tube current may change to the modified tube current immediately on application of the current modifier. In other examples, the current modifier may be applied to the tube current over a finite time interval. Particularly, the tube current may be ramped up/down to the modified tube current over the finite time interval, i.e., the tube current in the radiation source may be gradually changed until it becomes or is equal to the modified tube current.

As will be expanded on later, the current modifier may typically be applied (or at least start to be applied) to the tube current at a particular (e.g., target) time. This may correspond to a time immediately after the determination to apply the current modifier to the tube current, or a time later than this.

Once application of the current modifier to the tube current is completed, the tube current may stay as/at the modified tube current for an indefinite period of time. Particularly, the tube current may remain as/at the modified tube current until a further current modifier is applied at a later time (thereby further modifying the tube current).

As another example, once/after the current modifier is applied to the tube current (or a predetermined time interval after the current modifier is applied to the tube current) the (modified) tube current may return to the value of the tube current prior to application of the current modifier. As yet another example, after applying the current modifier, the tube current may return to following a predetermined (time-dependent) tube current, i.e., change to a tube current as given by the predetermined tube current at a present time.

Whether application of the current modifier comprises increasing or deceasing the tube current using the current modifier may typically depend on whether the count metric is considered to be above or below the predefined count range.

For example, when the predefined count range is defined by a (single) threshold, and being below the threshold is considered to be outside of the predefined count range, then applying the current modifier would comprise increasing the tube current using the current modifier. Thus, the current modifier may be set to a positive value for addition to the tube current, or to a factor greater than 1 for multiplication with the tube current. Alternatively, if being above the threshold is considered to be outside of the predefined count range, then applying the current modifier would comprise decreasing the tube current using the current modifier. Thus, the current modifier may be set to a negative value for addition to the tube current (or a positive value for subtraction from the tube current), or to a factor less than 1 for multiplication with the tube current.

Similarly, where the predefined count range is defined by a predefined lower count limit and a predefined upper count limit, applying the current modifier comprises increasing the tube current (using the current modifier) in response to the count metric being below the predefined lower count limit, and decreasing the tube current in response to the count metric being above the predefined upper count limit.

Application of the current modifier may thus act to bring the count metric (or more accurately count metrics for later instances of CT projection data) closer to the predefined count range, e.g., back within the predefined count range. Specifically, through adjusting the tube current, the amount of radiation received by the detector when acquiring later instances of CT projection data may be preferably modified (compared to if the tube current had stayed the same) such that the count metrics for said later instances of CT projection data are closer to the predefined count range.

In some examples, the magnitude/value of the current modifier is dependent on a difference between the count metric and the predefined count range, e.g., how much the count metric would have to be changed to bring it within the predefined count range. Particularly, where the predefined count range is defined by one or more thresholds or limits, the current modifier may be responsive to the difference between the count metric and one of said thresholds or limits, e.g., the threshold/limit closest to the count metric.

For example, where the predefined count range is defined by a predefined lower count limit (and a predefined upper count limit), the current modifier may be responsive to the difference between the count metric and the predefined lower count limit. It will be appreciated that this will only be the case in response to the count metric being below the predefined lower count limit, and thus being desirable for the count metric to move closer to the predefined lower count limit. Conversely, in response to the count metric being above the predefined upper count limit, the current modifier may be responsive to the difference between the count metric and the predefined upper count limit.

The current modifier may be proportional to this determined difference. Particularly, the determined difference is representative of a difference in counts. Thus, the current modifier may be determined through multiplying the determined difference by an appropriate scaling or conversion factor to convert the determined difference to a current difference. Accordingly, the current modifier may correspond to a change in current predicted to provide a desired change in counts.

The current modifier may be set such that the predicted change in counts is equal to the difference in counts represented by the determined difference (e.g., equal to the determined difference). This effectively corresponds to the minimum change in counts required to shift the count metric to within the predefined count range.

As another example, the current modifier may be set such that the predicted change in counts is more or less than the (minimum) change in counts required to shift the count metric to within the predefined count range. This may be modified through increasing or decreasing the scaling/conversion factor, or by introducing a further scaling factor when determining the current modifier.

Particularly, the magnitude of the current modifier relative to the size of the difference between the count metric and the predefined count range may be set based on a preferred rate of change of the tube current.

The above examples may be appropriate in cases where the current modifier comprises a current offset to be added or subtracted from the tube current.

As another example, where the current modifier comprises a scaling factor, the current modifier may be responsive to a percentage difference between the determined difference and the count metric. Particularly, said percentage difference may be converted to a multiplier (e.g., a scaling factor) required to shift the count metric to within the predefined count range. The current modifier may then be proportional to this multiplier, e.g., equal to the multiplier, a predetermined proportion of the multiplier, etc.

As previously mentioned, applying 240 the current modifier to the tube current may comprise applying the current modifier at a target time. Particularly, as performance of the monitoring process 205 may take a finite time, the time at which the monitoring process 205 reaches step 240 (and thus the earliest time at which the current modifier may be applied to the tube current) may not coincide with an appropriate time for applying the current modifier.

To provide more context, as the current modifier is determined based on the (present) instance of CT projection data, the current modifier is specific to the projection of the target volume that provided the instance of CT projection data. In other words, the current modifier is specific to the particular projection angle (or range of projection angles) used to acquire the instance of CT projection data.

However, due to the rotational motion of the radiation source, the projection angle of the radiation source at the time of reaching step 240 may not be the same as the projection angle associated with the current modifier. Therefore, applying the current modifier at this time may not be as effective as if it had been applied when acquiring the instance of CT projection data, e.g., as the projection of the target volume may be different and thus have different tube current requirements. It may therefore be desirable to apply the current modifier at a later (i.e., target) time, e.g., when the projection of the target volume is similar to that embodied in the instance of CT projection data.

According to a preferred example, the target time may be associated with a time at which the radiation source has rotated 180° relative to an angular position of the radiation source when the instance of CT projection data was obtained by the CT scanner. In other words, the target time may be associated with (e.g., equal to) a time of acquisition of a further instance of CT projection data whose associated projection angle is 180° different to the projection angle associated with the present instance of CT projection data.

This approach takes advantage of the similarity between projections of the target volume when taken from opposing sides of the CT scanner. Particularly, projections taken 180° apart are considered effectively equivalent due to being formed from similar paths through the target volume. Put another way, for the same target volume, attenuation of the beam (of radiation) is similar for projections taken 180° apart.

Thus, applying the current modifier for an instance of CT projection data acquired at a projection angle 180° later compared to the present instance of CT projection data is considered effectively equivalent to if the current modifier had been applied when acquiring the present instance of CT projection data. Accordingly, the current modifier may be applied at a target time associated with the acquisition of this later instance of CT projection data. For example, the target time may correspond to a time at which acquisition of the later instance of CT projection data begins, or a time slightly before acquisition of the later instance of CT projection data. Specifically, the target time may be determined based on a rotation speed of the CT scanner.

As a more general example, the target time may be associated with a time at which the radiation source has completed one or more 180° rotations relative to the projection angle associated with the instance of CT projection data, e.g., 360°, 540°, etc. Particularly, projections of the target volume are considered effectively equivalent from each of these angular positions. However, this equivalence/similarity may become less true/accurate for larger rotations dependent on an accompanying longitudinal displacement of the target volume with each full rotation of the CT scanner.

It will be appreciated that, between the determination to apply the current modifier and the actual application of the current modifier, further instances of CT projection data may have been acquired and processed using a monitoring process. Accordingly, before applying the current modifier, determinations to apply further current modifiers may also have been made. Thus, a series of current modifiers may be queued and applied in sequence according to their associated target times. **In** such examples, the magnitude of each current modifier may be controlled based on the knowledge that other current modifiers may be applied immediately afterwards. This approach helps to prevent overshooting or excessive offsetting of the tube current.

Prior to applying 240 the current modifier, the monitoring process 205 may comprise a step 235 of determining whether the modified tube current is within a predefined current range. It will be appreciated that the modified tube current in this context corresponds to the tube current that will be present in the radiation source after applying the current modifier in step 240. Thus, at the time of performing step 235, the tube current has not been changed to the modified tube current, but rather is still the same tube current as was present in the radiation source at the start of the present iteration of the monitoring process 205 (assuming the tube current has not been changed by other processes, e.g., other current modifiers). Step 235 therefore comprises checking whether the tube current after applying the current modifier will be within a predefined current range.

The predefined current range may typically correspond to a preferred or acceptable current range or window for the tube current, e.g., for meeting certain safety or instrumentation standards or requirements. Thus, it may be undesirable for the tube current to be outside of this range.

The monitoring process 205 may only be allowed to progress to step 240 in response to the modified tube current being within the predefined current range (i.e., a positive determination in step 235). Conversely, in response to the modified tube current being outside the predefined current range, step 240 may not be performed, i.e., the current modifier may not be applied to the tube current. Thus, in response to a negative determination in step 235, the current/present iteration of the monitoring process 205 may end.

The monitoring process 205 comprises a number of further steps for controlling the tube current in response to the count metric being within the predefined count range (i.e., a positive determination in step 230). Of course, in alternative embodiment, the monitoring process may end in response to the count metric being within the predefined count range, i.e., it may be determined that a current modifier does not need to be applied to the tube current.

The monitoring process 205 further comprises a step 250 of determining, responsive to the count metric being within the predefined count range, whether the tube current is different to a predetermined tube current.

In this context, the predetermined tube current may typically correspond to (e.g., comprise) a predetermined time-dependent tube current for use in a tube current modulation protocol. Particularly, the predetermined tube current may be determined responsive to a predetermined model of the target volume (e.g., based on a CT scout scan). Thus, the predetermined tube current may describe/indicate a preferred tube current for each time/moment/acquisition of the CT scan, e.g., for achieving a preferred image quality and/or signal-to-noise ratio in the resulting CT image.

While the count metric is within the predefined count window (and thus while the amount of radiation received by the detector is at an acceptable level) it may be preferable for the tube current to follow/match the predetermined tube current. Thus, in response to the tube current being different to the predetermined tube current (i.e., a positive determination in step 250), the monitoring process 205 progresses to a step 255 of applying a second current modifier to the tube current.

Where the predetermined tube current is a predetermined time-dependent tube current, the tube current being different to the predetermined tube current will be understood to mean that the tube current is different to the value of the predetermined tube current at the time of acquisition of the present instance of CT projection data.

For the avoidance of doubt, the second current modifier is different to the (first) current modifier applied in step 240. Particularly, the second current modifier is proportional to the difference between the tube current and the predetermined tube current. For example, the second current modifier may be equal to the difference between the tube current and the predetermined tube current. As another example, the second current modifier may be determined by multiplying the difference between the tube current and the predetermined tube current by a scaling factor, e.g., such that the second current modifier is less than the difference between the tube current and the predetermined tube current.

It will be appreciated that steps 250 and 255 may themselves constitute or enable a tube current modulation protocol. Particularly, the tube current may be controlled to follow the predetermined tube current by (iteratively) performing steps 250 and 255 in each iteration of the monitoring process 205. However, in response to the count metric being outside the predefined count range, the tube current modulation protocol may be overridden (i.e., steps 250 and 255 not performed) and a current modifier may be applied, thereby deviating the tube current away from the predetermined tube current. Once the count metric returns to being within the predefined count range, the tube current modulation protocol may be resumed (i.e., performing steps 250 and 255) and the tube current may resume following the predetermined tube current.

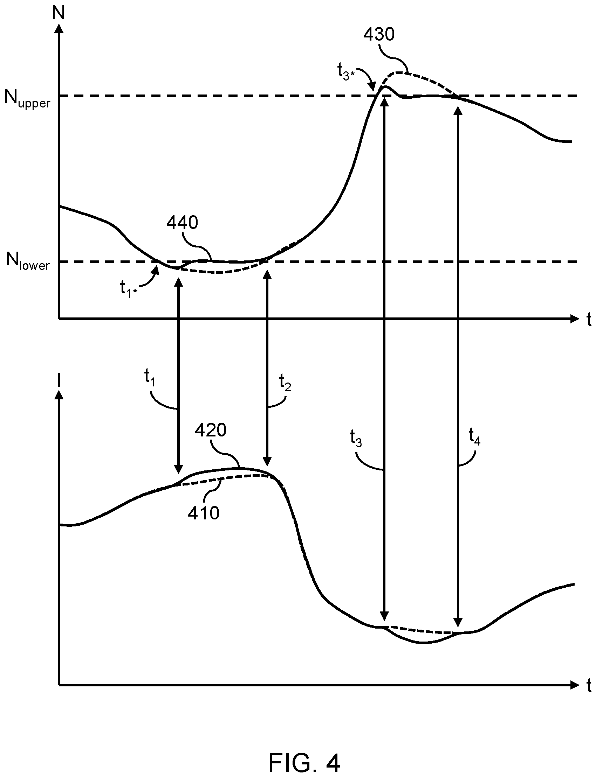

This process is conceptualized in Fig. 4, which illustrates variations in a count metric N and a tube current I as a function of time t. A predefined count range is defined by a predefined lower count limit N_{lower} and a predefined upper count limit Nᵤₚₚₑᵣ. Dashed line 410 depicts a predetermined tube current, and dashed line 430 depicts variations in the count metric N if the tube current I were to conform to the predetermined tube current. Meanwhile, lines 420 and 440 depict the actual behavior of the tube current I and count metric N when the proposed method 200 of Fig. 2 is applied.

In this context, the count metric N corresponds to a time-series of count metrics, each determined from a respective instance of CT projection data acquired over the time period depicted in Fig. 4.

Before a time t_{1*}, the count metric N is within the predefined count range. Thus, the tube current I follows/tracks the predetermined tube current, e.g., through a monitoring process iteratively applying a second current modifier to the tube current. At time t_{1*}, the count metric N falls below the predefined lower count limit N_{lower}. This may be due to the predetermined tube current not being appropriate or favorable for the part of the target volume being imaged at time t_{1*}. Thus, a current modifier is applied to the tube current to increase the count metric. Particularly, the current modifier is applied at a target time t₁. Thus, between times t_{1*} and t₁, the count metric N continues to fall below the predefined lower count limit N_{lower} but moves towards the predefined lower count limit N_{lower} after time t1.

Once the count metric N reaches the predefined lower count limit N_{lower}, the monitoring process returns to iteratively applying a second current modifier to the tube current so that it tracks the predetermined tube current. However, before time t₂, this results in the count metric N falling back below the predefined lower count limit N_{lower}, e.g., due to the predetermined tube current still being unfavorable. Thus, the count metric N may oscillate around the predefined lower count limit N_{lower} until the predetermined tube current becomes favorable for the part of the target volume being imaged. This is seen at time t₂ where the tube current I returns to tracking the predetermined tube current and the count metric N consistently stays above the predefined lower count limit N_{lower}.

Similar behavior is exhibited later between times t_{3*} and t₄ in response to the count metric N rising above the predefined upper count limit Nᵤₚₚₑᵣ.

This process may behave similar to (or take advantage of) a PID-type feedback loop. Particularly, the magnitude of each current modifier may be proportional to one or more differences (e.g., between the count metric and a predefined threshold/limit, between the tube current and a predetermined tube current, etc.) and one or more rates of change of said differences.

Returning to Fig. 2, the monitoring process further comprises a step 260 of determining, responsive to the count metric being within the predefined count range, whether the count metric is different to a predetermined count metric.

In this context, the predetermined count metric may typically be representative of a preferred or expected number of counts to be received by the detector, e.g., for achieving a preferred image quality and/or signal-to-noise ratio in the resulting CT image. Furthermore, the predetermined count metric may be a predetermined time-dependent count metric representative of a preferred or expected number of received counts at different times of a CT scan. The predetermined count metric may be determined responsive to a predetermined model of the target volume (e.g., based on a CT scout scan) in addition to a predetermined tube current, thereby defining an expected amount of radiation transmitted through the target volume at different times of a CT scan.

While the count metric is within the predefined count window (and thus while the amount of radiation received by the detector is at an acceptable level) it may be preferable for the count metric to follow/match the predetermined count metric. Thus, in response to the count metric being different to the predetermined tube current (i.e., a positive determination in step 260), the monitoring process 205 progresses to a step 265 of applying a third current modifier to the tube current.

Where the predetermined count metric is a predetermined time-dependent count metric, the count metric being different to the predetermined count metric will be understood to mean the count metric is different to the value of the predetermined count metric at the time of acquisition of the present instance of CT projection data.

For the avoidance of doubt, the third current modifier is different to the (first) current modifier and the second current modifier. Particularly, the third current modifier is proportional to the difference between the count metric and the predetermined count metric. For example, the third current modifier may be determined by multiplying the difference between the count metric and the predetermined count metric by a scaling/conversion factor, thereby converting the determined difference to a current difference.

It will be appreciated that steps 260 and 265 may themselves constitute or enable a tube current modulation protocol. Particularly, the tube current may be controlled so that the count metric from each instance of CT projection data follows the predetermined count metric, e.g., by (iteratively) performing steps 250 and 255 in each iteration of the monitoring process 205. However, in response to the count metric being outside the predefined count range, the tube current modulation protocol may be overridden (i.e., steps 260 and 265 not performed) and a current modifier may be applied. Once the count metric returns to being within the predefined count range, the tube current modulation protocol may be resumed (i.e., performing steps 250 and 255).

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

Functions implemented by a processor may be implemented by a single processor or by multiple separate processing units which may together be considered to constitute a "processor". Such processing units may in some cases be remote from each other and communicate with each other in a wired or wireless manner.

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

A single processor or other unit may fulfill the functions of several items recited in the claims. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

The mere fact that certain measures are recited in mutually different dependent claims does not indicate that a combination of these measures cannot be used to advantage.

In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A computer-implemented method (200) of modifying a tube current (I) of a radiation source (115) for a computer tomography, CT, scanner (110) during acquisition of a series of instances (120) of CT projection data of a target volume (330) using the CT scanner, the computer-implemented method comprising, for each instance of CT projection data of a first set of the series of instances of CT projection data:
receiving (210) the instance of CT projection data from the CT scanner;
processing (220) the instance of CT projection data to determine a count metric (N) responsive to a number of counts in the instance of CT projection data;
determining (230) whether the count metric is within a predefined count range; and
responsive to the count metric being outside the predefined count range, applying (240) a current modifier to the tube current, wherein applying the current modifier to the tube current comprises increasing or decreasing the tube current using the current modifier.

2. The computer-implemented method of claim 1, wherein:
the predefined count range is defined by a predefined lower count limit and a predefined upper count limit; and
applying the current modifier comprises:
in response to the count metric being below the predefined lower count limit, increasing the tube current using the current modifier; or
in response to the count metric being above the predefined upper count limit, decreasing the tube current using the current modifier.

3. The computer-implemented method of claim 2, wherein the current modifier is determined responsive to:
in response to the count metric being below the predefined lower count limit, a difference between the count metric and the predefined lower count limit; or
in response to the count metric being above the predefined upper count limit, a difference between the count metric and the predefined upper count limit.

4. The computer-implemented method of any one of claims 1 to 3, further comprising, prior to applying the current modifier to the tube current:
determining (235) whether a modified tube current, being the tube current after applying the current modifier, is within a predefined current range; and
responsive to the modified tube current being outside the predefined current range, not applying the current modifier to the tube current.

5. The computer-implemented method of any one of claims 1 to 4, wherein applying the current modifier to the tube current comprises applying the current modifier at a target time.

6. The computer-implemented method of claim 5, wherein the target time is associated with a time at which the radiation source has rotated 180° relative to an angular position of the radiation source when the instance of CT projection data was obtained by the CT scanner.

7. The computer-implemented method of any one of claims 1 to 6, wherein the count metric is equal to a number of counts in a predetermined portion of the instance of CT projection data, said predetermined portion having been obtained by a predetermined subset (S₁, S₂, S₃) of detector elements (118) of a detector (117) of the CT scanner.

8. The computer-implemented method of claim 7, wherein:
the predetermined subset of detector elements corresponds to a specific subset of detector elements for the instance of CT projection data predicted to obtain CT projection data of a target region (335) within the target volume; and
the predetermined subset of detector elements is determined responsive to a predetermined model of the target volume and a projection angle (*ϕ*) associated with the instance of CT projection data.

9. The computer-implemented method of claim 8, wherein the predetermined model of the target volume is determined responsive to a CT scout scan of the target volume.

10. The computer-implemented method of any one of claims 8 or 9, further comprising:
responsive to the count metric being within the predefined count range, determining (250) whether the tube current is different to a predetermined tube current, wherein the predetermined tube current is determined responsive to the predetermined model of the target volume; and
responsive to the tube current being different to the predetermined tube current, applying (255) a second current modifier to the tube current, wherein the second current modifier is proportional to a difference between the tube current and the predetermined tube current.

11. The computer-implemented method of any one of claims 8 to 10, further comprising:
responsive to the count metric being within the predefined count range, determining (260) whether the count metric is different to a predetermined count metric, wherein the predetermined count metric is determined responsive to a predetermined tube current and the predetermined model of the target volume; and
responsive to the count metric being different to the predetermined count metric, applying (265) a third current modifier to the tube current, wherein the third current modifier is proportional to a difference between the count metric and the predetermined count metric.

12. The computer-implemented method of any one of claims 1 to 11, wherein the CT scanner is a photon-counting CT scanner.

13. A computer program product comprising computer program code means which, when executed on a computing device having a processing system (130), cause the processing system to perform all of the steps of the method according to any one of claims 1 to 12.

14. A processing system (130) for modifying a tube current (I) of a radiation source (115) for a computer tomography, CT, scanner (110) during acquisition of a series of instances (120) of CT projection data of a target volume (330) using the CT scanner, the processing system being configured to, for each instance of CT projection data of a first set of the series of instances of CT projection data:
receive (210) the instance of CT projection data from the CT scanner;
process (220) the CT projection data to determine a count metric (N) responsive to a number of counts in the CT projection data;
determine (230) whether the count metric is within a predefined count range; and
responsive to the count metric being outside the predefined count range, apply (240) a current modifier to the tube current, wherein applying the current modifier to the tube current comprises increasing or decreasing the tube current using the current modifier.

15. A CT system (100) comprising:
the processing system of claim 14; and
the CT scanner.
